# EUROPEAN PATENT APPLICATION

(11) **EP 1 627 610 A1**
(43) Date of publication of application: **22.02.2006**
(21) Application number: 04019888.9
(22) Date of filing: 21.08.2004
(51) Int. Cl.: A61B 18/20

(54) **Drug delivery throug application in nails**

(71) Applicant: TLT Medical Ltd, 4143 Reinach, BL (CH)
(72) Inventor: Henrich, Werner, 4102 Binningen (CH)

(57) **Abstract**

A method for delivering a pharmaceutical composition to a human or animal nail, said method comprising forming several partial orifices in a nail with an appropriate device and applying a pharmaceutical composition in the orifices. Preferably the device comprises a laser programmed in a way to drill arrays of partial orifices where each partial orifices can be drilled with a single laser shot or, optionally with several laser shots. Preferably the partial orifices have similar depths across the nail. The method of the invention allows for the continuous treatment of diseases over a period of time with a minimum of patient discomfort combined with a high degree of efficacy.

## Description

The present invention relates to an improved method for delivering drugs or other agents with pharmacological or physiological action to a human or animal, said method comprising forming several partial orifices in a nail of the human or animal.

The nail plate is thick, hard, dense, and represents a barrier for drugs or other agents to be able to penetrate in a required quantity. Although nail material is similar to the stratum cornea of the skin, being derived from epidermis, it is composed primarily of hard keratin, which is highly disulfide-linked, and is approximately 100-fold thicker than stratum cornea.

According to the PCT/EP0/108558 application it has been found that nail diseases like onychomycosis can be successfully treated by forming with a laser one or more small orifices, into a nail plate, and applying an antifungal, e.g. a terbinafine containing composition to the nail. In order to secure a sufficient penetration of the drug into the deeper layers of the nail and in the nail bed an orifice described in that patent application means any small orifice or depression that penetrates 80 to 100% of the nail plate, preferably 90 to 99%.

According to the present invention it has now been found that the use of orifices between 10 to 80% of the nail plate provides a sufficient diffusion of the drug through the nail, and therefore, an unexpected high efficacy. This finding allows a significant improvement of the method described in PCT/EPO/108558.

For example the risk of inducing pain when drilling an orifice into or too close to the nail bed by e.g. heat or damage of nerve cells, is avoided. This improved method, in turns, simplifies the process and the architecture of the device for drilling orifices with no necessity for means e.g. a photoacoustic sensor, to assure that the drilling stops in time before reaching soft tissue.

Partial orifices according to the present invention means any small orifice or depression that penetrates between 15 to 80% of the nail plate, preferably 40 to 70%.

The partial orifices in the nail plate are formed preferably by means of a laser-based device comprising a laser which is used to form numerous orifices in the nail within a short period of time.

The laser device is programmed in order to allow drilling of orifices that penetrate 15 to 80% of the nail plate. In a most simple alternative the drilling would be between 0.2 and 0.8 mm and preferably 0.4 to 0.7 mm deep. In this way no individual measurement of nail thickness or a feedback system based on a photoacoustic sensor (PCT/EPO/108558) would be necessary. Preferably all orifices will have the same depth.

To control the depth of the individual orifices the laser power of a given pulse may be pre-calibrated with respect to the voltage, current and pulse duration of the laser power supply to determine the photoablation rate. Another simple way to control the depth of the orifices is to determine the depth of an orifice for a fixed voltage, current and pulse duration in the power supply for a single shot (e.g. 0.1 mm/laser shot). Once this information is known the user determines how many shots are needed to drill orifices of a given depth (e.g. 5 shots would be needed if the desired depth is 0.5 mm with a 0.1 mm depth/laser shot). Preferably all orifices will be drilled with identical conditions to ensure that all orifices have approximately the same depth depending on the usual shot-to-shot laser power fluctuation and variability in the nail water content and composition.

The diameter of the orifices are preferably from 1 µm (micrometer) to 1000 µm (= 1mm), most preferably from 50 µm to 250 µm. The orifices are preferably of cylindrical or conical shape.

Typically 100 to 1000 orifices/cm² of nail may be formed, preferably about 400 orifices/cm².

Photoablation refers to the melting and explosion of tissues and is achieved by pulsed laser irradiation of a selected wavelength, power and pulse duration according to the thermal, mechanical and spectral characteristics of the nail of interest, which may be a finger or toe human nail. The deposited electromagnetic energy is almost entirely transformed into mechanical energy and the illuminated region is ejected in the form of debris escaping the orifice at ca. 1'000 m/s. In a preferred photoablation process, the debris rapidly removes the deposited energy from the nail therefore the irradiated nail is not heated minimizing thus discomfort.

Any laser may be used provided it is capable of inducing ablation by rising the temperature in the illuminated region above 100°C. The laser may be selected from a Erbium (Er):YAG laser, a Nd:YAG laser, a OPO laser, a Ho:YAG laser, a CO₂ laser, a UV laser, or an excimer laser. A suitable UV laser is a nitrogen laser. Suitable excimer lasers include a Kr laser and a XE laser. Most preferably, the laser is a Er:YAG (λ = 2.94 µm) laser, a Ho:YAG laser (λ = 2.1 µm), or a CO₂ gas laser (λ = 10.6 µm) because water strongly absorb electromagnetic radiation of such wavelengths. A combination of lasers may also be used.

In one embodiment of the invention small orifices are formed with a single laser shot of ca. 150 mJ of enery delivered in about 200 µs of duration. In a second embodiment of the invention small orifices are formed with multiple laser shots of ca. 50 mJ of energy in about. 100 µs of duration each at a repetition rate of 20 Hz. The number of laser shots needed to drill each orifice in the second embodiment could be 2 to 30 laser shots, most preferably between 2 to 10 if the photoablation laser operates a low repetition rate (i.e. less than 100 Hz) such as those pumped by flash lamps. However, if the photoablation laser is of the new generation pumped by laser diodes that can be operated at repetition rates in the KHz range having less power per laser, the orifices could be drilled with up to 3000 laser shots.

In addition to the laser, its electronic power supply and its cooling system, the laser-based device may include one of the following elements:
(a) a separate multifunctional toe or finger docking element designed in such way that it prevents laser light to escape from the apparatus into the free-space once it is attached to the laser-based device. The docking element may be constructed with a material having good acoustic properties to reduce the noise caused by the photoablation process. The docking element may also be used for relative z-positioning of the nail vs. the laser beam waist in order to avoid a sophisticated active autofocus system. In this configuration the relative z-positioning of the nail vs. the laser beam waist is realized by forcing the nail against a mechanical stop using a spring loaded clamp to compensate for the shape diversity in fingers or toes. The finger and toe docking element may also have a transparent optical window to allow the visualization of the nail by means of the monitoring video element (c) and to keep the optical components of the laser-based device near the nail clean. The docking element may have a toe or finger support to fasten the toe or nail (e.g. by a spring loaded clamp or a band) leaving the nail plate uncovered for the laser exposure.
   When the docking element (a) is attached to the laser-based device, it may open
(b) a mechanical shutter or optical diaphragm of the laser-based device for enhanced optical safety which is otherwise closed when e.g. the device is warming up. In such geometrical configuration, the optical path of the photoablation laser beam finishes at the nail. In this way the photoablation laser beam is always jacketed inside the device for enhanced patient and user safety. The shutter could be placed in any place inside the laser-based device but most preferably at the end of the laser based device where the docking element attaches but it can also be placed right after the output coupler mirror of the laser head.
   Furthermore, the nail plate may be visualized by means of
(c) a video camera or charged coupled device (CCD) camera or similar device inside the laser-based device to monitor the nail plate on the screen of a computer to determine the area of the nail to be treated.
(d) a vacuum system connected to the docking element (a) to remove the photoablation debris to keep the optical components near the nail being treated clean during the whole photoablation procedure.
(e) a computer controlled xy translation stage module or scanner to position the laser beam in the desired area of the nail which is fixed to the laser-based apparatus by means of the docking element (a). Preferably, the toe or finger may be fixed and the laser beam is positioned on the desired part of nail plate surface for photoablation purposes. In such embodiment the xy module could also be a galvano-optical positioning element. Alternatively, the docking element (a) may be mounted on this translation stage so that the laser beam is fixed and the toe or finger being treated moves. Another possibility is that both, the toe or finger being treated, fixed to the docking element (a), moves into a given axis (e.g. x), while the laser beam moves the y axis;
(f) a low power targeting laser emitting in the visible part of the spectrum or, a Light Emitting Diode (LED), to anticipate the spot in the nail to be irradiated by the photoablation and targeting laser or LED.
(g) mirrors which may be used to coaxially mix the photoablation laser beam with the light from the targeting laser or LED (f); and to divert the image of the nail onto the video monitoring video element (c). Most preferably these mirrors are dichoric mirrors;
(h) an optical focussing element comprising at least one lens that can change its distance with respect to the nail plate dorsal surface to ensure that the waist of the focussed laser beam is properly positioned with respect to the nail in spite of its non-planar surface before the laser is fired;
(i) a computer to fulfil many tasks including: the monitoring of the nail plate by means of a video camera or charged coupled device camera (c) to design of the specific laser treatment according to the degree of infection of the nail to be treated, to control the positioning of the photoablation laser beam waist precisely where the orifices are to be formed, to control the different laser parameters (e.g. the firing of the laser when the desired position of the xy translation stage (e) has been reached, or the laser power and pulse duration, to control the number of shots per orifice etc.. The laser beam inside the laser-based device can propagate in the air or, in parts of its path inside
(j) an optical fiber, and
(k) an optical element to multiplex the laser beam(s) (e.g. a diffractive optical element such as Dammann grating) to make more than one orifice (e.g. an array of equally spaced orifices) at the same time thereby avoiding to make the orifices one-by-one in a subsequent mode.

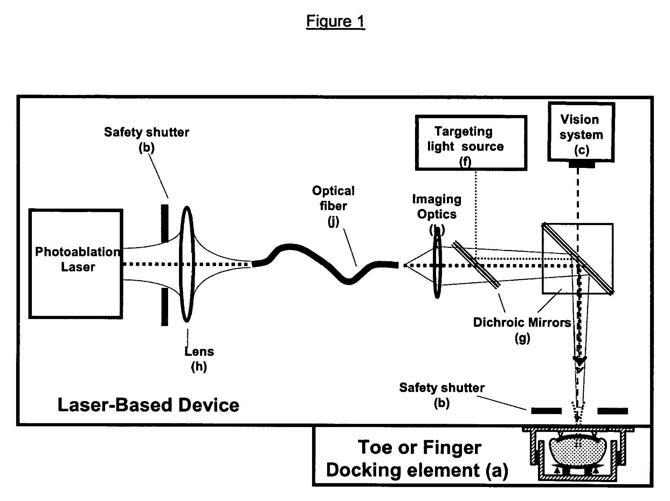

The invention will now be described in more detail with particular reference to the accompanying drawings of which
FIG. 1 is a general schematic representation of a laser-based device with the docking element (a) being attached.

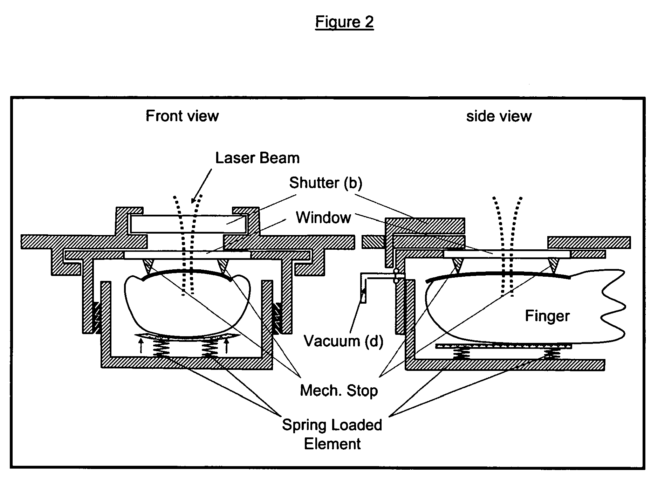

FIG. 2 is a general schematic representation of the toe or finger docking element (a).

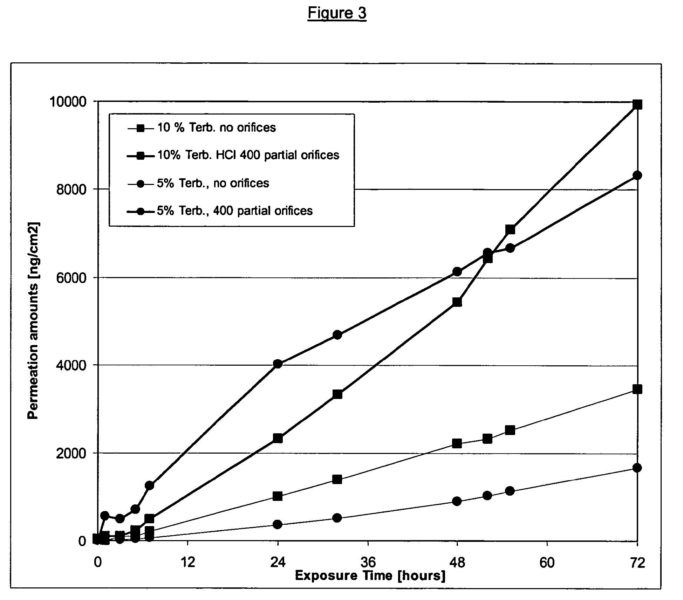

FIG 3 depicts the in-vitro permeation fluxes in [ng/cm²] of terbinafine containing formulations in nails having 400 orifices/cm² with similar depths of 0.5 mm each (amounting to 50% to 70% of the total thickness of the nail plate depending on the region) and in nails which were not treated. The terbinafine concentration of the formulations were 5% and 10% (w/w) in the vehicle described in Table 3. It is readily apparent that the terbinafine permeation fluxes in the laser pre-treated nails are three to four times higher than the permeation fluxes of untreated nails. The assays used to obtain the reported results and the details of the laser pre-treated nails are described in Example 1.

In one aspect of the present invention the new method of treatment using partial orifices in the nail is used for treating infections of the nail as e.g. onychomycosis or nail psoriasis.

In a preferred embodiment onychomycosis is treated through the use of an antifungal agent e.g. terbinafine administered into partial orifices drilled by an appropriate laser device. In another embodiment nail psoriasis is treated with the method of the invention and the use of e.g. corticosteroids.

The method of treatment of the present invention can be combined with other treatments of the same disease e.g. an oral treatment with the aim to reduce side effects or with a conventional topical treatment in order to increase its efficacy. The method of treatment could also be combined with the debriding of the infected part of the nail by standard medical procedures before the present treatment is applied only to the healthy (i.e. not infected) part of the nail.

In a further aspect the method of the invention provides a controlled release of a pharmaceutical composition. The controlled release of the pharmaceutical composition may be used to administer a pharmaceutical composition systemically and to act at a body side remote from the nail.

The agent with a pharmacological or physiological activity according the invention comprises at least one active ingredient. For the purpose of the invention, "active ingredient" means all substances that produce a pharmaceutical, therapeutic, physiological or prophylactic effect. Active ingredients may include without limitation, antifungals, photosensitizers, androgens, estrogens, nonsteroidal anti-inflammatory agents, antihypertensive agents, analgesic agents, antidepressants, antibiotics, anticancer agents, anesthetics, antiemetics, antiinfectants, contraceptives, antidiabetic agents, steroids, anti-allergy agents, anti-migraine agents, agents for smoking cessation, anti-obesity agents and vaccines.

Examples of active ingredients include the following: acebutolol, acetylcysteine, acetaminophen, acetylsalicylic acid, acyclovir, alprazolam, alfacalcidol, allantoin, allopurinol, ambroxol, amikacin, amiloride, aminoacetic acid, amiodarone, amitriptyline, amlodipine, amoxicillin, ampicillin, ascorbic acid, astemizole, atenolol, beclomethasone, benserazide, benzalkonium hydrochloride, benzocaine, betamethasone, bezafibrate, biotin, biperiden, bisoprolol, bromazepam, bromhexine, bromocriptine, budesonide, bufexamac, buflomedil, bupivacaine, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, selegiline, chloramphenicol, chlorhexidine, chlor-pheniramine, chlortalidone, choline, cyclosporin, cilastatin, cimetidine, ciprofloxacin, cisapride, cisplatin, clarithromycin, clavulanic acid, clomidine, clomipramine, clonazepam, clonidine, clotrimazole, codeine, cholestyramine, cromoglycic acid, cyanocobalamin, cyproterone, desogestrel, dexamethasone, dexpanthenol, dexamethasone, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinylestradiol, etoposide, Eucalyptus globulus, famotidine, felodipine, fenofibrate, fenoterol, fentanyl, flavin mononucleotide, fluconazole, flunarizine, fluorouracil, fluoxetine, flurbiprofen, folic acid, folinic acid, furosemide, gallopamil, gemfibrozil, gentamicin, Gingko biloba, glibenclamide, glipizide, clozapine, Glycyrrhiza glabra, griseofulvin, haloperidol, heparin, hyaluronic acid, hydrochlorothiazide, hydrocodone, hydrocortisone, hydromorphone, ipratropium hydroxide, ibuprofen, imipenem, indomethacin, insulin, iohexol, iopamidol, isosorbide dinitrate, isosorbide mononitrate, isotretinoin, ketotifen, ketoconazole, ketoprofen, ketorolac, labetalol, lactulose, levocarnitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylprednisolone, metoclopramide, metoprolol, miconazole, midazolam, minocycline, minoxidil, misoprostol, morphine, multivitamin mixtures and combinations and mineral salts, N-methylephedrine, naftidrofuryl, naproxen, neomycin, nicardipine, nicergoline, nicotinamide, nicotine, nicotinic acid, nifedipine, nimodipine, nitrazepam, nitrendipine, nitroglycerine, nizatidine, norethisterone, norfloxacin, norgestrel, nortriptyline, nystatin, ofloxacin, omeprazole, ondansetron, pancreatin, panthenol, pantothenic acid, paracetamol, penicillin G, penicillin V, phenobarbital, pentoxifylline, phenoxymethylpenicillin, phenylephrine, phenylpropanolamine, phenytoin, piroxicam, polymyxin B, povidone-iodine, pravastatin, prazepam, prazosin, prednisolone, prednisone, prilocaine, progesterone, propafenone, propranolol, proxyphylline, pseudoephedrine, pyridoxine, quinidine, ramipril, ranitidine, reserpine, retinol, riboflavin, rifampicin, rutoside, salbutamol, salcatonin, salicylic acid, scopolamine, simvastatin, somatotropin, sotalol, spironolactone, sucralfate, sufentanil, sulbactam, sulfamethoxazole, sulfasalazine, sulpiride, sumatriptan, tamoxifen, tegafur, teprenone, terbinafine, terazosin, terbutaline, terfenadine, testosterone, tetracaine, tetracycline, theophylline, thiamine, ticlopidine, timolol, tranexamic acid, tretinoin, triamcinolone acetonide, triamterene, trimethoprim, troxerutin, uracil, valproic acid, vancomycin, verapamil, vitamin A, vitamin C, vitamin E, and zidovudine. A combination of active ingredients may also be used. Preferably the active ingredient is selected from a photosensitizer. Most preferably the active ingredient is terbinafine.

Vaccine may include without limitation Smallpox, Rabies, Plaque, Diphtheria, Dertussis, Tuberculosis, Tetanus, Yellow Fever, Polio Vaccine, Measales, Mumps, Rubella, Hepatitis B, Hepatitis C, Haemophilus influenza Type B, Japanese Encephalitis, Biomanguinhos, Human Influenza Typ B (Hib), cancer.

The vaccines are preferably vaccines which require multiple inoculation to achieve protective titers such as Hepatitis B and hepatitis C.

More preferably the vaccines are vaccines which require long contact with dendritic cells to achieve a cytotoxic T-cell response such as Hepatitis B, HIV, human Papilloma virus (HPV) and cancer. The nail bed has a high concentration of Langerhans cells that stimulate the immune response. A robust immune response may be obtained by the slow release of vaccines by the method of the invention.

The cancer vaccines may be made of whole cancer cells or of substances contained by the tumor. Preferably the cancer vaccines are selected from the group consisting of whole cancer cells, peptides, proteins, dendritic cells, gangliosides, heat-shock proteins, viral and bacterial vectors and nucleic acids.

The amount of active ingredient in the pharmaceutical composition according to the present invention may vary from 0.1% to 100 % weight of active ingredient to weight of the total weight of the pharmaceutical composition. Preferably the active ingredient is present in an amount of from 0.1 % to 99%, preferably from 20% to 80%, more preferably 10 to 40, weight percent. The dose of active ingredient and exposure time depends on the number, diameter and shape of the orifices and on the nature and severeness of the disease to be treated. In a most preferred embodiment the pharmaceutical composition contains terbinafine in 10 to 30% and is used for the treatment of onychomycosis.

Additional components may be used in the pharmaceutical compositions or applied directly to a partial orifice prior to or following the addition of the pharmaceutical composition to the orifice. Such additional ingredients include natural and/or artificial ingredients which are commonly used to prepare pharmaceutical compositions. Examples of additional ingredients include surfactants, diluents, binders, disintegrating agents, anti caking agents, vitamins, botanicals, supplements, herbs, minerals, trace elements, amino acids (e.g., L-tryptophan), fibers, enzymes, fillers, buffers, colorants, dyes, antioxidants, preservatives, electrolytes, glidants, disintegrates, lubricants, and carrier materials (e.g. Aloe Vera). A combination of additional ingredients may also be used. Such ingredients are known to those skilled in the art.

More generally all formulation types which have been applied to the skin or hair may, with adaptation to the specific active ingredient, be used in the method of the invention.

The pharmaceutical composition of the transungual formulation to be used in conjunction with the present method may be in the form of a varnish, lacquer, liquid, semi-solid, solid, solution, gel, emulsion, or powder.

Most preferred vehicles to be used in the transungual formulations are a lacquer described in USP 4'957'730 having the composition given in Table1.:

**Table 1**

| **Excipients** | **Composition (wt%)** |
|---|---|
| Ethylacetat | 30.00 |
| Isopropanol | 30.00 |
| Gantrez Polymer ES | 40.00 |
| Butylhydroxitoluene | 0.00 |
| Total | 100.00 |

where Gantrez is the trademark name of butyl monoester of poly[methylvinylether/maleic acid].

Another suitable transungual vehicle to use in the transungual formulation is the nail varnish described in USP 6'319'509 and EP 515'312 having the composition given in Table 2.

**Table 2**

| **Excipients** | **Composition (wt%)** |
|---|---|
| lsopropylmiristat | 2.100 |
| Triacetin | 2.100 |
| Eudragit RS 100 | 26.300 |
| Butylhydroxytoluol | 0.021 |
| Water purified | 5.260 |
| Ethanol 94% | 64.219 |
| Total | 100.000 |

A gel described in US 6,211,250 / EP 944,398 is also a suitable vehicle for transungual formulations having the composition given in Table 3.

**Table 3**

| **Excipients** | **Composition (wt%)** |
|---|---|
| Klucel MF | 2.5 |
| Dermacryl 97 | 5.0 |
| Miglyol 812 | 5.0 |
| Ethanol 94% G/G | 87.5 |
| Total | 100.0 |

### Example 1.

The proof-of-concept demonstrating the virtues of the invention for onychomycosis is based on a series of in-vitro nail permeation assays using human cadaveric toe nails using standard Franz-type diffusion cells (described in J. T. Franz, Dermatology, 184, p-18, 1992) originally intended for transdermal studies, which were modified to accept human cadaveric toenails. Franz cells comprises two chambers separated by the nail plate. The radiolabelled [¹⁴C]-terbinafine containing transungual formulations were applied in the donor upper chamber and the receptor chamber is filled with a buffer to simulate the human physiological conditions. To account for the fact the nails are hard and curved, wetted nails are clamped between two flexible flat silicone polymer disks. The temperature of the cells was kept a 30°C using a circulating water bath. Magnetic stirrer bars were used to ensure receptor uniformity throughout an experiment.

The nail permeation assays consisted of exposing the dorsal side of the nail plates to the investigated formulations while monitoring the appearance the amount of radiolabelled [¹⁴C]-terbinafine on the ventral side by LC-MS in the receptor fluid chamber of the Franz cells. In all cases a single dose of 100 µL was applied in the nail dorsal side. 100 µL of buffer were taken for each time point for LSC measurement which was replaced by buffer in order to keep the total buffer volume constant. The sampling time points were 0, 1, 3, 5, 7, 24, 32, 48, 52, 55 and 72 hours.

Human cadaveric thumb toenails used in the permeation assays were harvested from diseased adults (age > 50 years old) in order to match thickness and consistence of the nails of the patient age group having a higher incidence to onychomycosis. Nails were randomly selected for the permeation assays and thawed at room temperature for at least 1 hand soaked in de-ionized water for a few hours before the adhering skin and soft tissue from the ventral side was removed with scissors and scalpel. Once clean, nails were punched into disks of diameters of 13 to 15 mm to fit into the Franz-type diffusion cells.

The density of the partial orifices in the laser pre-treated nails used in the assays reported in Figure 3 is 400 orifices/cm². All orifices have similar depths of 0.5±0.1 mm each amounting to 50% to 70% of the total thickness of the nail plate depending on the region. The diameter of the orifices was 250±50 µm drilled, in all cases, with five shots of 50±10 mJ of energy per laser pulse lasting 80±10 µs each.

While the invention has been described with particular reference to certain embodiments thereof, it will be understood that changes and modifications may be made by those of ordinary skill within the scope and spirit of the following claims.

## Claims

1. A method for delivering a pharmaceutical or physiological composition to a human or animal, said method comprising forming several partial orifices in a nail of the human or animal and applying the composition in the orifices..

2. A method according to claim 1 wherein the orifices are formed by means of a device which secures a penetration of the orifices at a depth between 15 and 80% of the nail plate.

3. A method according to claims 1 and 2 wherein the device secures a penetration of 40% to 70% of the nail plate.

4. A method according to claim 1 wherein the device secures a penetration of 0.1 to 0.8 mm into the nail plate.

5. A method according to claims 1 to 4 where the device is a laser.

6. The method according to claim 1 wherein the composition comprises at least one pharmaceutical or physiological active ingredient present in 0.1 % to 99% based on the total weight of the pharmaceutical composition.

7. The method according to claim 6 wherein the active ingredient is terbinafine present in 10 to 30%, based on the total weight of the pharmaceutical composition.

8. The method according to claims 1 to 7 wherein the diameter of the orifice is from 1 µm to 1000 µm.

9. The method according to claim 8 wherein the diameter of the orifice is from 50 µm to 200 µm.

10. A method according to claims 1 to 9 where the density of orifices is 50 to 1000 orifices/cm².

11. A method according to claims 1 to 10 where each partial orifice of predetermined depth is formed by a single laser shot.

12. A method according to claims 1 to 10 where each partial orifice of predetermined depth is formed by multiple laser shots.

13. A method according to claim 12 where each partial orifice of predetermined depth is formed through 2 to 10 laser shots.

14. A method according to claims 1 to 13 where all partial orifices to be drilled in a given nail have approximately the same predetermined depth.

15. A device suitable for the performance of the method according to claims 1 to 14 comprising a laser providing electromagnetic radiation of a given power and/or pulse duration which secures the drilling of partial orifices of a predetermined depth into the nail plate.

16. The device according to claim 15 comprising a toe or finger docking element.

17. A device according to claim 16 wherein the toe or finger docking element has a support to fasten the toe or finger to be treated, a vacuum connexion, a light jacket and an optical window.

18. The device according to claims 15 to 17 comprising an optical shutter.

19. Use of the method according to the claims 1 to 14 for the treatment of onychomycosis.

20. Use of the method according to the claims 1 to 14 for the treatment of nail psoriasis.
